# EUROPEAN PATENT SPECIFICATION

(11) **EP 2 349 343 B1**
(45) Date of publication and mention of the grant of the patent: **18.09.2013**
(21) Application number: 09759869.2
(22) Date of filing: 30.09.2009
(51) Int. Cl.: A61K 47/48, A61P 35/00

(54) **POLYMERIC COMPOSITION WITH SYNERGISTIC EFFECT IN TREATMENT OF TUMOUR DISEASES**
ZUSAMMENSETZUNG VON POLYMEREN MIT SYNERGISTISCHEM EFFEKT BEI DER BEHANDLUNG VON TUMORERKRANKUNGEN
COMPOSITION POLYMÈRE À EFFET SYNERGIQUE POUR TRAITER LES MALADIES TUMORALES

(30) Priority: 23.10.2008 CZ 20080661
(43) Date of publication of application: 03.08.2011
(73) Proprietor: Zentiva, K.S., 102 37 Praha 10 (CZ)
(72) Inventor: ETRYCH, Tomas, 252 10 Klinec (CZ); ULBRICH, Karel, 140 00 Prague 4 (CZ); HOVORKA, Ondrej, 130 00 Praha 3 (CZ); RIHOVA, Blanka, 140 00 Prague 4 (CZ)
(74) Representative: Jirotkova, Ivana
(86) International application number: PCT/CZ2009/000115
(87) International publication number: WO 2010/045896

(56) References cited:
- WO-A1-2008/034391
- WO-A2-03/053473
- KOVAR LUBOMIR ET AL: "The same drug but a different mechanism of action: Comparison of free doxorubicin with two different N-(2-hydroxypropyl)methacrylamide copolymer-bound doxorubicin conjugates in EL-4 cancer cell line" BIOCONJUGATE CHEMISTRY, vol. 18, no. 3, May 2007 (2007-05), pages 894-902, XP002586382 ISSN: 1043-1802 cited in the application
- RHOVA B ET AL: "Doxorubicin bound to a HPMA copolymer carrier through hydrazone bond is effective also in a cancer cell line with a limited content of lysosomes" JOURNAL OF CONTROLLED RELEASE, ELSEVIER, AMSTERDAM, NL LNKD- DOI:10.1016/S0168-3659(01)00320-0, vol. 74, no. 1-3, 6 July 2001 (2001-07-06) , pages 225-232, XP004297527 ISSN: 0168-3659 cited in the application
- KOVAR M ET AL: "HPMA copolymers containing doxorubicin bound by a proteolytically or hydrolytically cleavable bond: comparison of biological properties in vitro" JOURNAL OF CONTROLLED RELEASE, ELSEVIER, AMSTERDAM, NL LNKD- DOI:10.1016/J.JCONREL.2004.07.015, vol. 99, no. 2, 30 September 2004 (2004-09-30), pages 301-314, XP004569550 ISSN: 0168-3659 cited in the application
- SIROVA M ET AL: "TREATMENT WITH HPMA COPOLYMER-BASED DOXORUBICIN CONJUGATE CONTAINING HUMAN IMMUNOGLOBULIN INDUCES LONG-LASTING SYSTEMIC ANTI-TUMOUR IMMUNITY IN MICE" CANCER IMMUNOLOGY AND IMMUNOTHERAPY, SPRINGER-VERLAG, BERLIN, DE LNKD- DOI:10.1007/S00262-006-0168-0, vol. 56, no. 1, 1 January 2007 (2007-01-01), pages 35-47, XP019458905 ISSN: 0340-7004 cited in the application
- ULBRICH K ET AL: "HPMA copolymers with pH-controlled release of doxorubicin - In vitro cytotoxicity and in vivo antitumor activity" JOURNAL OF CONTROLLED RELEASE, ELSEVIER, AMSTERDAM, NL LNKD- DOI:10.1016/S0168-3659(02)00348-6, vol. 87, no. 1-3, 21 February 2003 (2003-02-21), pages 33-47, XP004412737 ISSN: 0168-3659 cited in the application
- ETRYCH T ET AL: "SYNTHESIS OF HPMA COPOLYMERS CONTAINING DOXORUBICIN BOUND VIA A HYDRAZONE LINKAGE. EFFECT OF SPACER ON DRUG RELEASE AND IN VITRO CYTOTOXICITY" MACROMOLECULAR BIOSCIENCE, WILEY VCH VERLAG, WEINHEIM, DE LNKD- DOI:10.1002/1616-5195(20020101)2:1&LT,43:: AID-MABI43&GT,3.0.CO,2-8, vol. 2, no. 1, 1 January 2002 (2002-01-01) , pages 43-52, XP008076237 ISSN: 1616-5187 cited in the application
- CHYTIL P ET AL: "Properties of HPMA copolymer-doxorubicin conjugates with pH-controlled activation: Effect of polymer chain modification" JOURNAL OF CONTROLLED RELEASE, ELSEVIER, AMSTERDAM, NL LNKD- DOI:10.1016/J.JCONREL.2006.06.028, vol. 115, no. 1, 28 September 2006 (2006-09-28), pages 26-36, XP024957636 ISSN: 0168-3659 [retrieved on 2006-09-28]

## Description

### Technical Field

The invention relates to a polymeric composition consisting of a mixture of two polymeric drugs (doxorubicin conjugates) providing high-effective treatment of tumour diseases. Use of the polymeric composition is focused on a targeted therapy of tumour diseases, in particular solid tumours, in human medicine.

### Background Art

Chemotherapy of tumour diseases targets the use of drugs with minimized side effects on healthy tissues and the maximum specific effect on tumour tissue or even particular tumour cells. Using of polymeric carriers in preparation of new generations of cancerostatics offers both a possibility to achieve targeted transport of a cancerostatic drug to a tumour tissue and controlled activation of the carrier-bound drug at the point of the required effect, i.e. in tumour tissue or even inside a tumour cell. It was shown that an appropriate molecular weight of a polymeric carrier can provide preferential deposition of the polymeric drug in tumour tissue in the whole range of solid tumours (so-called EPR effect) [Maeda et al. 2000]. Releasing of the cytostatic from the polymeric carrier can be resolved through a biodegradable link used for binding of the drug to the polymer. Degradation and thus release of the drug can occur via either enzymatic and/or chemical hydrolysis.

An important group of these drugs includes polymeric drugs prepared based on copolymers of *N*-(2-hydroxy-propyl)methacrylamide (HPMA). A range of polymeric cancerostatics was developed, prepared on the basis of copolymers of *N*-(2-hydroxy-propyl)methacrylamide (HPMA) providing both passive [Kope 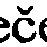 ek et al. 2000, 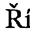 íhová et al. 2001, Lammers et al. 2005 and 2006, Kissel et al. 2001, Ulbrich et al. 2000] and active [ 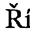 íhová et al. 2000, Jelínková et al. 2003, 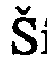 írová et al. 2007, Ulbrich et al. 2004,] directing of such cancerostatic to the tumour. The first such polymeric cancerostatic substance providing passive directing of cancerostatic to solid tumours, which was tested clinically, was a conjugate of HPMA copolymer with doxorubicin (DOX) called PK1 [Duncan et al. 1985, Vasey et al. 1999, Julian et al, 1999]. DOX in this drug was bound to the polymeric carrier using an oligopeptide sequence degradable in biological environment by lysosomal enzymes. It has been demonstrated recently [Hovorka et al. 2002] that such a polymeric drug penetrates very fast to tumour cells, binds to cell organelle membranes such as endoplasmic reticulum, Golgi apparatus, lysosomes and endosomes and causes destruction of the cell which mostly occurs due to necrotic changes in the cell. Although a significant anti-tumour effect of such a drug in patients with various types of tumours with simultaneous decrease in side toxicity was demonstrated [ 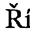 íhová et al. 2003a, 2003b and 2005], therapeutic effect was not so convincing that this drug could be used commercially.

In 1985, a structurally very similar drug was patented yet carrying in its structure units enabling targeted directing of polymeric cancerostatic to membrane receptors of tumour cells [Duncan et al. 1985]. For this directing some amino sugars, hormones and in particular antibodies were used. Anti-tumour effect of a conjugate of HPMA copolymer, doxorubicin and Intraglobin or Endobulin antibodies was also verified clinically [et al. 2003, 2005]. In vivo experiments in mice showed a high anti-tumour activity of other conjugates directed by monoclonal antibodies. In all cases DOX was bound to a polymeric carrier through an enzymatically cleavable oligopeptide sequence. An overview of so-far achieved results in this area is elaborated very well in a G.S: Kwon's monograph and J. Kope 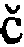 ek's et al. work [Kope 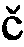 ek et al. 2000, Kwon 2005] and other [Kim 2003].

Recently, some studies dealing with effects of polymeric drugs have been published, in which the cancerostatic doxorubicin was bound to a polymeric carrier prepared on the basis of HPMA copolymers through a hydrolytically unstable hydrazone bonds (HYDRAZONE) [Etrych et al. and 2002, íhová et al. 2001, Ulbrich et al. 2003, 2004] and these substances were patented in several patent applications [Ulbrich et al. CZ 293886, Etrych et al. PA of 2005 and 2006, Chytil a et al. PA of 2006]. Such drugs showed significant reduction of side, in particular toxic effects on healthy organism with simultaneous significant increase in anti-tumour effect compared to commonly used cytostatics [ 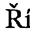 íhovà et al. 2001, Ková 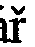 et al. 2004, Hovorka et al. 2002] even compared to the PK1 conjugate, both in a non-directed form and a form directed by an antibody. It was shown that in the case of HYDRAZONE the mode of effect of the polymeric cancerostatic is different from effects of conjugates of PK1 type and tumour cells die particularly due to apoptosis [Hovorka et al. 2002, Ková 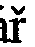 et al. 2004, Ková 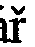 et al. 2007].

The subject of this invention is a composition consisting of two types of the above-mentioned polymeric drugs differing in mode of effect on tumour cells, mixed in ratios resulting in a synergistic effect of both types of drugs and thus in a particularly significant anti-tumour effect with very low non-specific drug toxicity. Causing of an autostimulating anti-tumour effect is another significant effect accompanying the therapy with the composition.

### Disclosure of Invention

The essence of the invention is a composition consisting of two components - polymeric conjugates of doxorubicin intended for treatment of tumour diseases possessing significant cytotoxic anti-tumour activity and simultaneously an autostimulating anti-tumour effect.

**The first component** forming the composition is a HPMA copolymer (PK1), in which the principle lies in that DOX is linked to the polymeric carrier through the enzymatically cleavable oligopeptide GlyPheLeuGly sequence. The polymeric chain consists of 50 - 1000 monomer units consisting of 80 - 99 mol% of N-(2-hydroxy-propyl)methacrylamide (HPMA) units, 1 - 5 mol% of methacryloylated oligopeptide (GlyPheLeuGly) terminated with an amidic bond of bound doxorubicin, 0 - 10 mol% of methacryloylated oligopeptide (GlyPheLeuGly) terminated with a carboxyl group, 0 - 10 mol% of methacryloylated oligopeptide (GlyPheLeuGly) terminated with the 2-hydroxypropylamide group, or 0.5 - 10 mol% of methacryloylated oligopeptide units (GlyPheLeuGly) terminated with a molecule of human non-specific (HuIg, Endobulin, Intraglobin) or monoclonal antibody (Erbitux, Herceptin).

**The second component** is a HPMA copolymer (HYDRAZONE, H), the principle of which lies in DOX linked to the polymeric carrier through a spacer containing a pH-sensitive hydrolytically labile hydrazone bond. The copolymer consists of 30 - 3500 monomer units connected in a linear or branched polymeric chain formed by 60 to 98.5 % of HPMA units, and further contains 1 to 20 % units of methacryloylated hydrazones of α-amino acids, ε-amino acids, aromatic amino acids or oligopeptides terminated with the doxorubicin molecule, 0.5 to 15 % units of hydrazides of methacryloylated hydrazones of α-amino acids, ε-amino acids, aromatic amino acids or oligopeptides or their sodium salts. The conjugate may have bound 0.5 to 5 % of units of methacryloylated α-amino acids, ε-amino acids, aromatic amino acids or oligopeptides terminated with a molecule of immunoglobulin or specific monoclonal antibody.

Preferably, the composition of the invention can be composed of two components, the first of which can be illustrated by formula I 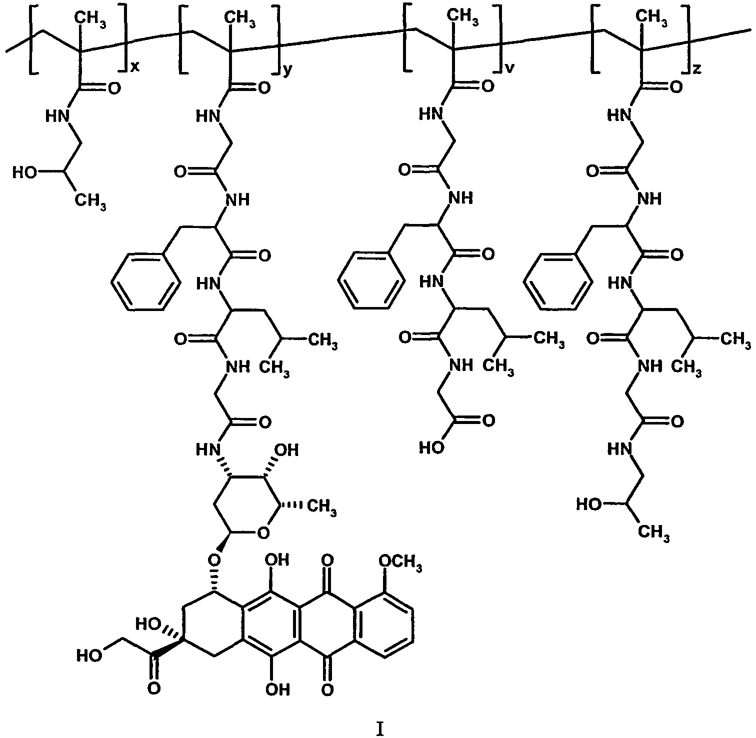 in which x, y, v and z take the values of: x = 40 to 990; y = 1 to 50; v = 0 to 100 and z = 0 to 100.

The second component can be illustrated by formula II: 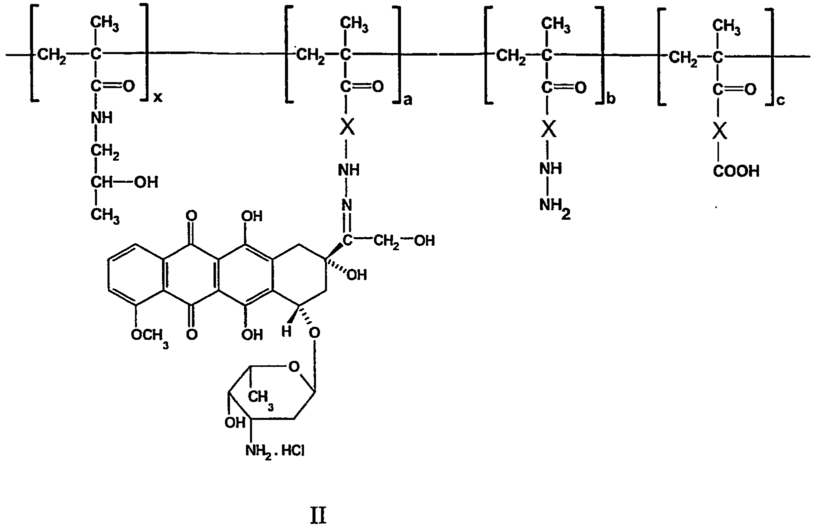 in which parameters x, a, b, c take the values of: x = 20 to 3000; units A, a = 1 to 700; units B, b = 1 to 450 and units C, c = 1 to 450; X represents a link formed by α-amino acid, ε-amino acid, aromatic amino acid or oligopeptide, optionally in the form of their sodium salts.

The second component can also be represented by a polymeric micelle according to CZ PV 2006/207 [Chytil 2006]. The scheme of the polymeric micelle can be illustrated by formula III

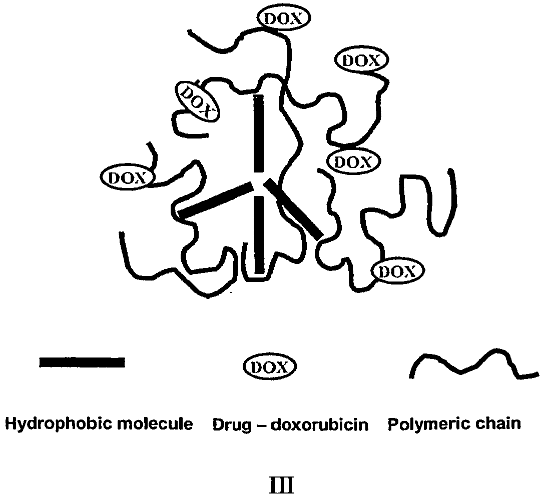

The hydrophilic surface of this micelle consists of a HPMA copolymer of type I or II. The hydrophobic component is aliphatic or cyclic carbohydrates or their derivatives.

To the main HPMA chain in both types of components side chains can be attached. Such a grafted copolymer is described in detail in CZ patent application PV 2006/592 [Etrych 2006]. Connection between the side and main chain is realized in a tumour cell through a degradable link.

Both the micelle arrangement or the arrangement in a grafted copolymer will enable better penetration of the conjugate to tumour tissue of solid tumours by the above-described EPR effect. Both types of conjugate degrade, along with concurrent release of the active ingredient, to lower polymeric units which are easily excreted from the body. Both effects are described in detail in respective CZ patent applications PV 2006/2007 and PV 2006/592.

According to this invention, the composition is characterized by the individual components (polymeric conjugates) being mixed in the product so that the resulting ratio of the weight of doxorubicin bound by amidic bond (PK) and that bound by hydrazone bond (H) is in a ratio of PK/H from 1 : 3 to 3 : 1, preferably in the ratio 1 : 2 (calculated as a ratio of the amount of the drug in the individual components of the system).

### Brief Description of Drawings:

Figure 1 illustrates survival of B/6 strain mice with inoculated T-cell lymphoma EL 4 (s.c.) treated with one dose of 15 mg DOX eq/kg of the composition (weight ratio of drug in PK 1/H 1:2), conjugate PK1 and Hydrazone conjugate administered by injection (IV) on Day 8 after inoculation of tumour cells.

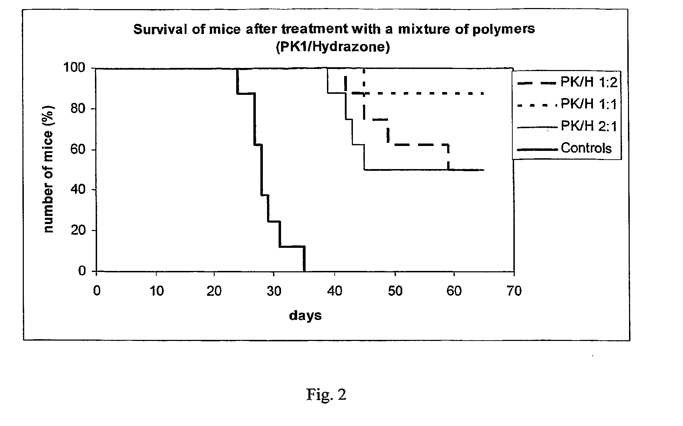

Figure 2 illustrates survival of C57BL/6 strain mice with subcutaneously inoculated T-cell lymphoma EL4 treated with one dose of 10 mg DOX eq/kg of the composition administered by injection (IV) on Day 8 after inoculation of tumour cells.

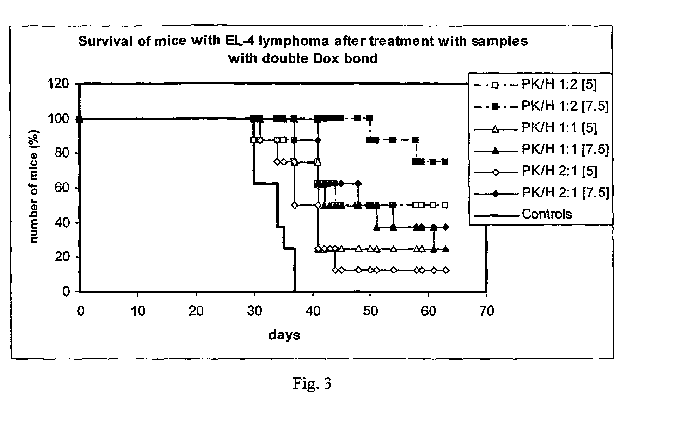

Fig. 3 illustrates survival of mice strain C57BL6 with s.c. inoculated T-cell lymphoma EL4 treated with one dose of 5 or 7.5 mg DOX eq/kg of the composition administered by injection (IV) on Day 9 after inoculation of tumour cells.

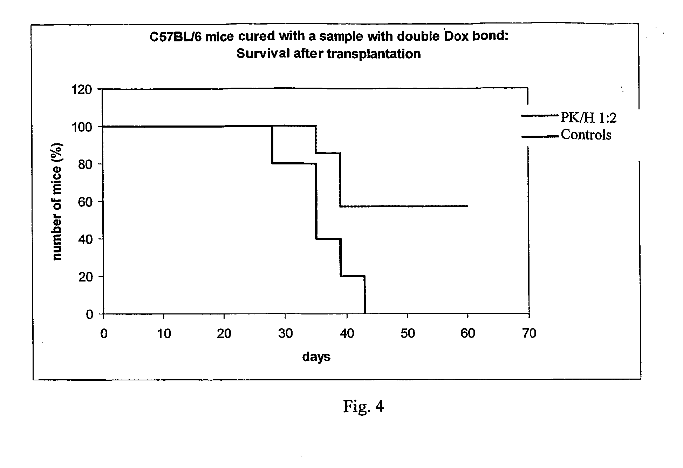

Fig. 4 illustrates survival of mice strain C57BL/6 cured of murine T-cell lymphoma EL 4 by administering the composition according to example 1 inoculated with a lethal dose of the same tumour and left without treatment.

### Examples:

In the examples the above-mentioned components labelled PK1 and Hydrazone were used with the following specification:
PK1 consisted of 95.2 mol % (x = 0.952) of units of N-(2-hydroxypropyl)methacrylamide (HPMA), 1.9 mol% methacryloylated oligopeptide (GlyPheLeuGly) terminated with an amidic bond of the bound doxorubicin (formula I) (y = 0.019), 2.9 mol% (z = 0.029) of methacryloylated oligopeptide (GlyPheLeuGly) terminated with 2-hydroxypropylamide group (formula III). The content of carboxyls was minimal (v∼0) and its content was within the measurement accuracy limits.
Hydrazone consisted of 94.2 mol % (x = 0.942) of HPMA units and also contains 3.8 mol % (a = 0.038) of units of methacryloylated hydrazone of 6-aminohexanoic acid terminated with a molecule of doxorubicin and 2 mol % (b = 0.02) of units of methacryloylated 6-aminohexanoic acid.

The content of units with carboxyl (c) was again within the measurement error limits.

### Example 1

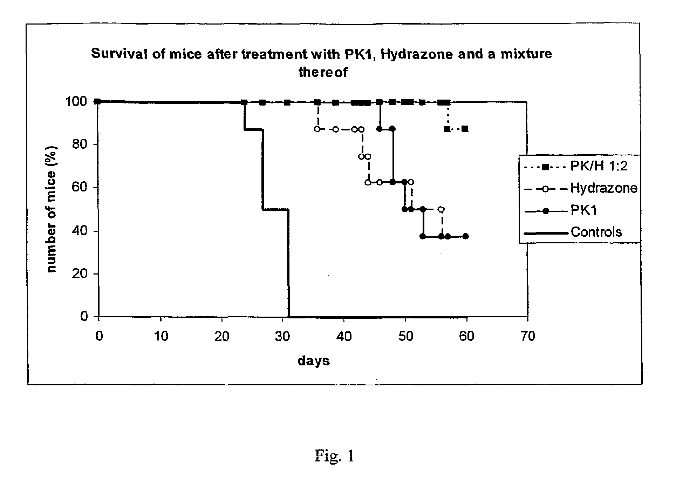

1x10⁵ cells of murine T-cell lymphoma EL 4 was inoculated subcutaneously to females of strain C57BL/6. After 8 days of incubation when the tumour become palpable (the size of 120 — 150 mm³), the animals were administered intravenously one dose of a solution of the composition in the ratio 1:2 of weight content of doxorubicin in conjugates PK1 and Hydrazone PK, a solution of PK1 conjugate and Hydrazone in 0.2 ml of saline so that the final concentration of doxorubicin corresponded always to the dose of 15 mg of DOX eq/kg. Saline was used as the control (0.2 ml). The product consisted of Hydrazone (M_{w} = 34.7 kDa, content of free hydrazide groups 2 % mol., content of DOX 13.5 % wt.) and conjugate PK1 (M_{w} = 25 kDa, content of GFLG sequences 4.9 % mol and content of DOX 6.6 % wt.). Growth of the tumour, weight of the mice and survival of the animals were observed. Compared to the control mice which died between days 24 to 31, in the treated mice growth of the tumour was inhibited, which was the most distinct after administration of the composition. By Day 73 the tumour was not detected in any surviving animals treated with the composition (7 long-tem surviving mice out of 8 treated mice). Efficiency of the treatment with conjugates PK1 alone and/or Hydrazone was considerably lower, which manifested in both less considerable inhibition of tumour growth and a lower number of surviving animals (3 surviving out of 8 treated), see Fig. 1. Side toxicity manifesting in a loss of weight was not seen with any of the conjugates or the composition.

### Example 2

1x10⁵ cells of murine T-cell lymphoma EL 4 was inoculated subcutaneously to females of strain C57BL/6. After 8 days of incubation when the tumour become palpable with the size of 100 - 150 mm³, the animals were administered one intravenous dose of a solution of the composition of three different constitutions (with different ratios of PK1 and Hydrazone mixed so that the resulting ratio of the drug in the product was - PK/H in ratios 2:1, 1:1 and 1:2) in 0.2 ml of saline so that the final concentration always corresponds to the total dose 10 mg DOX eq/kg. The composition consisted of Hydrazone (M_{w} = 34.7 kDa, content of free hydrazide groups 2 % mol., content of DOX 13.5 % wt) and conjugate PK1 (M_{w} = 25 kDa, content of GFLG sequences 4.9 % mol. and content of DOX 6.6 % wt). Compared to the control mice, which died between Days 24 to 35, growth of tumour in the treated mice was inhibited significantly and then mice were cured with efficiency depending on the product composition. With the composition of PK/H 1:1,7 out of 8 experimental animals survived in the long-term. With the composition of PK/H 1:2, 4 out of 4 mice survived in the long-term, likewise as with the ratio PK/H 2:1. With this lower dose it is not possible to cure any animals in the same therapy regime with the PK1 conjugate; the same efficiency of treatment with the Hydrazone conjugate can be achieved only with the dose 25 mg DOX eq/kg and higher.

### Example 3

A composition of the same constitution as that in Example 2 was applied to females of strain C57BL/6 carrying an inoculated tumour of murine T-cell lymphoma EL 4 (1x10⁵ cells) on Day 9 after inoculation in the amount 5 and 7.5 mg DOX eq/kg. Even this lower dose of the active drug, depending on the constitution and dose of the composition, resulted in long-term cure of significant per cent of treated animals (27-75 %). Like in Example 1 it is not possible to cure experimental animals with conjugate PK1 with such a low dose administered in the same treatment regime. To cure about 25 % of experimental animals the dose of 25 mg DOX eq/kg and higher must be used. The same efficiency of treatment with the Hydrazone conjugate (seven out of eight animals) can be achieved only with the dose of ca. 25 mg DOX eq/kg and higher.

### Example 4:

Females of mice inbred strain C57BL/6, cured by administrating the composition as mentioned in Example 1, were on Day 73 after administration of the first transplant of the murine T-cell lymphoma EL 4 re-transplanted with the lethal dose (1 x 10⁵ cells) of the same tumour cells and left without administration of the composition. The control group consisted of conventional mice to which the same dose of tumour cells was administered. Growth of tumour was observed and long-term survival was recorded (% of so-called LTS = long term survivors). While the control mice died between Days 28 and 43, 57 % of mice of the group cured administering of the composition according to Example 1 and re-transplanted with the same tumour cells survived for 60 and more days. This long-term surviving of the lethal dose of tumour cells without chemotherapy can be explained only by the fact that with the efficient treatment with the composition according to Example 1 the immune system is stimulated concurrently and protects efficiently the re-transplanted animals from a developing tumour.

### References:

R. Duncan, J.B. Lloyd, J. Kope 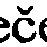 ek, P. Rejmanová, J. Strohalm, K. Ulbrich, B. 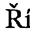 íhová, V. Chytrý, Synthetic Polymeric Drugs. Czech. PV 0095/1985
T. Etrych, M. Jelínková, B. 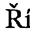 íhová, K. Ulbrich, New HPMA copolymers containing doxorubicin bound via pH sensitive linkage. Synthesis, in vitro and in vivo biological properties. J. Controlled Rel. 73, 89-102 (2001)
T. Etrych, P. Chytil, M. Jelínková, B. 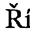 íhová, K. Ulbrich, Synthesis of HPMA Copolymers Containing Doxorubicin Bound via a Hydrazone Linkage. Effect of Spacer on Drug Release and in vitro Cytotoxicity. Macromolecular Biosci. 2, 43-52 (2002)
T. Etrych, K. Ulbrich, P. Chytil, M. Studenovský, M. Pechar, Způsob p 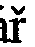 ípravy polymerních konjugátů doxorubicinu s pH- 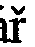 ízeným uvol 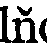 ováním lé 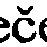 iva. CZ PV 524-2005
T. Etrych, P. Chytil, M. Pechar, M. Studenovský, B. 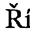 íhová a K. Ulbrich, Způsob p 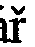 ípravy polymerních konjugátů doxorubicinu s pH- 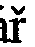 ízeným uvol 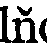 ováním lé 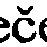 iva. CZ PV 2005-558
T. Etrych, K. Ulbrich: Způsob p 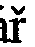 ípravy polymerních konjugátů doxorubicinu s pH- 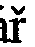 ízeným uvol 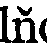 ováním lé 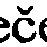 iva. Method of preparing polymeric conjugates of doxorubicin with pH-regulated release of the drug. CZ PV-2006-505
T. Etrych, P. Chytil, K. Ulbrich, T. Mrkvan, B. 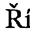 íhová, Roubované vysokomolekulární konjugáty doxorubicinu s protinádorovou aktivitou a způsob jejich výroby, PV 2006 - 592
O. Hovorka, T. Etrych, M. 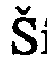 ubr, J. Strohalm, K. Ulbrich, B. 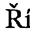 íhová, Differences in the Intracellular Fate of Free and Polymer-Bound Doxorubicin. J. Controlled Release 80, 101-117 (2002)
P. Chytil, T. Etrych, M. Hrubý, K. Ulbrich, B. 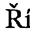 íhová: Micelární nosi 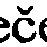 e lé 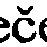 iv s protinádorovou aktivitou. CZ PV 2006-207
M. Jelínková, J. Strohalm, T. Etrych, K. Ulbrich, B. 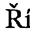 íhová, Starlike versus Classic Macromolecular Prodrugs: Two Different Antibody-Targeted HPMA Copolymers of Doxorubicin Studied In Vitro and In Vivo as Potential Anticancer Drugs. Pharm. Res. 20, 1556-1564 (2003)
J.A.Kim, Targeted Therapies for the treatment of cancer. Am. J. Surgery 186, 264-268 (2003)
M. Kissel, P. Peschke, V. 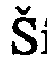 ubr, K. Ulbrich, J. Schuhmacher, J. Debus, E. Friedrich, Synthetic Macromolecular Drug Carriers: Biodistribution of Poly[N-(2-hydroxypropyl)methacrylamide] and its Accumulation in Solid Rat Tumors. PDA J. Pharm. Sci. Technol. 55, 191-201 (2001)
J. Kope 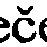 ek, P. Kope 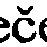 ková, T. Minko, Z. Lu, HPMA Copolymer-Anticancer Drug Conjugates: Design, Activity, and Mechanism of Action. Europ. J. Pharm. Biopharm. 50, 61 - 81 (2000)
M. Ková 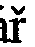 , L.Ková 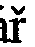 , V. 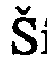 ubr, T. Etrych, K. Ulbrich, T. Mrkvan, J. Loucká and B. 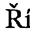 íhová, HPMA Copolymers Containing Doxorubicin Bound by Proteolytically or Hydrolytically Cleavable Bond: Comparison of Biological Properties In Vitro. J. Controlled Release 99,301-314 (2004)
L. Ková 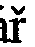 , J. Strohalm, P. Chytil, T. Mrkvan, M. Ková 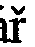 , O. Hovorka, K. Ulbrich, B. 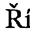 íhová,. The same drug but a different mechanism of action: comparison of free doxorubicin with two different N-(2-hydroxypropyl)methacrylamide copolymer-bound doxorubicin conjugates in EL-4 cancer cell line. Bioconjugate Chemistry, 18, 894-902 (2007)
G.S. Kwon, Polymeric Drug Delivery Systems, Series: Drugs and the Pharmaceutical Sciences, Vol. 148, Dekker, Marcel Incorporated, 2005
T. Lammers, R. Kühnlein, V. 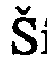 ubr, K. Ulbrich, G. Storm, P. Peschke, P. Huber, Effect of intratumoral injection on the biodistribution and therapeutic potential of HPMA copolymers. Neoplasia 8, 788 - 795 (2006)
T.Lammers, R. Kühnlein, M. Kissel, V. 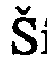 ubr, T. Etrych, R. Pola, M. Pechar, K. Ulbrich, G. Storm, P. Huber, P. Peschke: Effect of physicochemical modification on the biodistribution and tumor accumulation of HPMA copolymers. J. Controlled Release 110, 103-118 (2005)
H. Maeda, J. Wu, T. Sawa, Y. Matsumura, K. Hori, Tumor vascular permeability and the EPR effect in macromolecular therapeutics: a review. J Control Release 65, 271-284 (2000).
B. 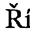 íhová, M. Jelínková, J. Strohalm, M. 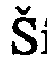 t'astný, M. Hovorka, D. Plocová, M. Ková 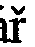 , L. Drábelová, K. Ulbrich, The Anti-proliferative effect of Lectin- and Anti-Thy-1.2 Antibody-targeted HPMA Copolymer-bound Doxorubicin on Primary and Methastatic Human Colorectal Carcinoma and on Human Colorectal Carcinoma Transfected with Mouse Thy-1.2 Gene. Bioconjugate Chemistry, 11, 664-673 (2000)
B. 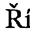 íhová, T. Etrych, M. Pechar, M. Jelínková, M. 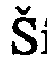 t'astný, O. Hovorka, M. Ková 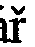 , K. Ulbrich, Doxorubicin Bound to a HPMA Copolymer Carrier Through Hydrazone Bond is Effective also in a Cancer Cell Line with a Limited Content of Lysosomes. J. Controlled Release 74, 225-232 (2001)
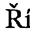 íhová, J. Strohalm, K. Kubá 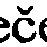 ková, M. Jelínková, L. Rozprimová, M. 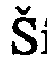 írová, D. Plocová, T.Mrkvan, M. Ková 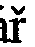 , J. Pokorná, T. Etrych, K. Ulbrich, Drug-HPMA-HuIg Conjugates Effective Against Human Solid Cancer. Advances in Experimental Medicine and Biology, Vol. 19. Polymer Drugs in the Clinical Stage, Maeda et al Eds., New York, 125 - 143 (2003a)
B. 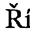 íhová, K. Kubá 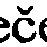 ková, Clinical Implications of N-(2-Hydroxypropyl)Methacrylamide Copolymers. Current Pharmaceut. Biotech. 4, 311-322 (2003b
B. 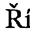 íhová, J. Strohalm, M. Ková 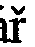 , T. Mrkvan, V. 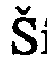 ubr, O. Hovorka, M. 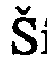 írová, L.Rozprimová, K. Kubá 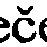 ková, K. Ulbrich, Induction of Systemic Antitumour Resistence with Targeted Polymers. Scandinavian J. Immunology 62, 100 - 105 (2005)
M, 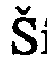 írová, J. Strohalm, V. 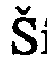 ubr, D. Plocová, T. Mrkvan, K. Ulbrich, B. 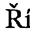 íhová, Treatment with HPMA copolymer-based doxorubicin conjugate containing human immunoglobulin induces systemic and long-lasting anti-tumor immunity in mice. Cancer Immunol. Immunother. 56, 35-47 (2007)
K. Ulbrich, V. 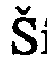 ubr, J. Strohalm, D. Plocová, M. Jelínková, B. 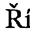 íhová, Polymeric Drugs Based on Conjugates of Synthetic and Natural Macromolecules I. Synthesis and Physicochemical Characterisation. J. Controlled Rel. 64, 63-79 (2000)
K. Ulbrich, T. Etrych, P. Chytil, M. Jelínková, B. 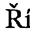 íhová, Antibody-Targeted Polymer-Doxorubicin Conjugates with pH-Controlled Activation. J. Drug Targeting, 12, 477-490 (2004)
K. Ulbrich, T. Etrych, P. Chytil, M. Jelínková, B. 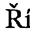 íhová, HPMA Copolymers with pH-Controlled Release of Doxorubicin. In vitro Cytotoxicity and in vivo Antitumor Activity. J. Controlled Release 87, 33-47 (2003)
K. Ulbrich, T. Etrych, P. Chytil, M. Pechar, M. Jelínková, B. 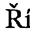 íhová, Polymeric Anticancer Drugs with pH-Controlled Activation. Int. J. Pharm. 277/1-2 67-72 (2004)
K. Ulbrich, T. Etrych, B. 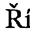 íhová, M. Jelínková, M. Ková 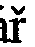 : pH Senzitivni polymerní konjugáty doxorubicinu pro cílenou terapii. CZ 293886, Europ. Pat. Appl. 06025316.8-1216

## Claims

1. A pharmaceutical composition having important cytotoxic anti-tumour activity and concurrent autostimulating anti-tumour effect, with the active ingredient doxorubicin, *wherein* the composition consists of two components, wherein:
a) the first component forming the composition is a copolymer of N-(2-hydroxypropyl)methacrylamide (HPMA) marked PK1, in which doxorubicin is linked to the polymeric carrier through enzymatically cleavable oligopeptide GlyPheLeuGly sequence, wherein the polymeric chain consists of 50 - 1000 monomer units consisting of 80 - 99 mol% of N-(2-hydroxy-propyl)methacrylamide (HPMA) units, 1 - 5 mol% of methacryloylated oligopeptide (GlyPheLeuGly) terminated with an amidic bond of bound doxorubicin, 0 - 10 mol% of methacryloylated oligopeptide (GlyPheLeuGly) terminated with a carboxyl group, 0 - 10 mol% of methacryloylated oligopeptide (GlyPheLeuGly) terminated with the 2-hydroxypropyl amide group and optionally 0.5 -10 mol% of units of a methacryloylated oligopeptide (GlyPheLeuGly) terminated with a molecule of a human non-specific antibody, such as HuIg, Endobulin, Intraglobin, or a monoclonal antibody such as Erbitux or Herceptin;
b) the second component is a HPMA copolymer marked H, in which doxorubicin is linked to the polymeric carrier through a spacer containing a pH-sensitive hydrolytically labile hydrazone bond, which consists of 30 - 3500 monomer units connected in a linear or branched polymeric chain formed by 60 to 98.5 % of HPMA units, and further contains 1 to 20 % units of methacryloylated hydrazones of α-amino acids, ε-amino acids, aromatic amino acids or oligopeptides terminated with a doxorubicin molecule, 0.5 to 15 % units of hydrazides of methacryloylated hydrazones of α-amino acids, ε-amino acids, aromatic amino acids or oligopeptides, or their sodium salts, and optionally 0.5 to 5 % units of methacryloylated α-amino acids, ε-amino acids, aromatic amino acids or oligopeptides terminated with a molecule of an immunoglobulin or a specific monoclonal antibody.

2. The composition according to claim 1, ***characterized in that*** the components are mixed so that the resulting dose of doxorubicin of the first component PK1 in which doxorubicin is bound by an amidic bond and the second component H in which it is bound by a hydrazone bond is in a weight ratio PK1/H1 : 3 to 3 :1.

3. The composition according to claim 2, ***characterized in that*** the components are in such a ratio that the resulting dose of doxorubicin PK1/H is in a weight ratio 1 : 2.

4. The composition according to any one of the preceding claims, ***characterized in that*** at least one of the components is formed by a system of polymeric micelles, the hydrophilic surface of which consist of a HPMA copolymer, while the hydrophobic core is formed by aliphatic C₉ to C₃₀ carbohydrates, or their derivatives, or is formed by cyclic or polycyclic C₆ to C₃₀ carbohydrates.

5. The composition according to claim 4, ***characterized in that*** the hydrophobic molecule is selected from the group consisting of acyls of C₁₀ to C₁₈ fatty acids, or unsaturated fatty acids or cholic, cholanic (5ß-cholane.24-oic) or 7-dehydrocholic acids.

6. The composition according to claim 4, ***characterized in that*** the hydrophobic molecule is selected from the group consisting of esters of cholesterol or 7-dehydrocholesterol, cholestanol, vitamin D or aliphatic alcohols with C₉ to C₁₈ chains.

7. The composition according to any one of claims 1 to 3, ***characterized in that*** the structure of at least one of the components consists of the main chain of HPMA carrying the active ingredient and of side chains of HPMA - grafts which may optionally also carry the active ingredient, said grafts being connected to the main chain through a bond cleavable in a tumour cell.

8. The composition according to claim 7, ***characterized in that*** the molecular weight of the component containing side chains amounts to 50 000 to 400 000 g/mol.

9. The composition according to claims 7 or 8, ***characterized in that*** the bonds linking the main and side chains are degradable either in an enzymatic or a reducing way.

## Patentansprüche

1. Pharmazeutische Zusammensetzung mit wichtiger zytotoxischen Anti-Tumor-Aktivität und gleichzeitiger selbststimulierenden Anti-Tumor Wirkung, mit der Wirkstoff Doxorubicin, wobei die Zusammensetzung aus zwei Komponenten besteht, wobei
a. die erste Komponente bildend die Zusammensetzung ein Copolymer von *N-*(2-Hydroxypropyl)methacrylamid (HPMA), PK1 bezeichnet, ist, in welchem Doxorubicin an den polymerem Träger durch eine enzymatisch spaltbare Oligopeptidsequenz GlyPheLeuGly verbunden ist, wobei die polymere Kette aus 50-1000 Monomereinheiten, bestehend aus 80-99 Mol-% N-(2-Hydroxypropyl)methacrylamid (HPMA)-Einheiten, 1-5 Mol-% von methacryloylierten Oligopeptids (GlyPheLeuGly), terminiert mit einer Amidbindung von verbundenen Doxorubicin, 0-10 Mol-% von methacryloylierten Oligopeptids (GlyPheLeuGly), terminiert mit einer Karboxylbindung, 0-10 Mol-% von methacryloylierten Oligopeptids (GlyPheLeuGly), terminiert mit der 2-Hydroxypropylbindung und gegebenenfalls 0,5-10 Mol-% von methacryloylierten Oligopeptids (GlyPheLeuGly), terminiert mit einem Molekül eines humanen nichtspezifischen Antikörper, wie Hulg, Endobulin, Intraglobin, oder eines monoklonalen Antikörper, wie Erbitux oder Herceptin, besteht;
b. die zweite Komponente ein Copolymer, H bezeichnet, ist, in welchem Doxorubicin an den polymerem Träger durch einen Spacer verbunden ist, enthaltend eine pH-empfindliche hydrolytisch labile Hydrazonbindung, welches aus 30-3500 Monomereinheiten, verbunden in einer linearen oder verzweigten Polymerkette aus 60-98,5 % HPMA-Einheiten gebildet, besteht, und ferner 1-20 % von Einheiten von methacryloylierten Hydrazonen von α-Aminosäuren, ε-Aminosäuren, aromatischen Aminosäuren oder Peptiden terminiert mit Doxorubicin-Molekül, 0,5-15 % von Einheiten von Hydraziden von methacryloylierten Hydrazonen von α-Aminosäuren, ε-Aminosäuren, aromatischen Aminosäuren oder Oligopeptiden oder deren Natriumsalzen, und gegebenenfalls 0,5-5 % von Einheiten von methacryloylierten α-Aminosäuren, ε-Aminosäuren, aromatischen Aminosäuren oder Oligopeptiden terminiert mit einen Molekül eines Immunglobulins oder eines spezifischen Antikörper, enthält.

2. Zusammensetzung nach Anspruch 1, **dadurch gekennzeichnet, dass** die Komponenten so gemischt werden, dass die resultierende Dosis von Doxorubicin der ersten Komponente PK1, in welcher Doxorubicin durch die Amidbindung, und der zweiten Komponente H, in welcher es durch die Hydrazonbindung verbunden ist, in einem Gewichtsverhältnis PK1/H von 1 : 3 bis 3 : 1 ist.

3. Zusammensetzung nach Anspruch 2, **dadurch gekennzeichnet, dass** die Komponenten in solchem Verhältnis sind, dass die resultierende Dosis von Doxorubicin PK1/H einem Gewichtsverhältnis von 1 : 3 bis 3 : 1 ist.

4. Zusammensetzung nach einem der vorhergehenden Ansprüche, **dadurch gekennzeichnet, dass** mindestens eine der Komponenten von einem System der polymerem Mizellen gebildet ist, dessen hydrophile Oberfläche aus einem HPMA-Copolymer besteht, während das hydrophobe Kern aus aliphatischen C₉-C₃₀ Kohlenwasserstoffen oder deren Derivaten, oder cyclischen oder polycyclischen C₆-C₃₀ Kohlenwasserstoffen gebildet ist.

5. Zusammensetzung nach Anspruch 4, **dadurch gekennzeichnet, dass** das hydrophobe Molekül aus der Gruppe, bestehend aus Acylen von C₁₀-C₁₈ Fettsäuren, oder ungesättigten Fettsäuren, oder Cholsäure, Cholansäure (5β-Cholan.24-Säure) oder 7-Dehydrocholsäure, ausgewählt ist.

6. Zusammensetzung nach Anspruch 4, **dadurch gekennzeichnet, dass** das hydrophobe Molekül aus der Gruppe, bestehend aus Estern von Cholesterol oder 7-Dehydrocholesterol, Cholestanol, Vitamin D oder C₉-C₁₈-kettigen aliphatischen Alkoholen, ausgewählt ist.

7. Zusammensetzung nach einem der Ansprüche 1 bis 3, **dadurch gekennzeichnet, dass** die Struktur von mindestens einer der Komponenten aus der Hauptkette von HPMA, tragend die Wirkstoff, und den Seitenketten von HPMA- Pfropfen, welche auch die Wirkstoff tragen können, wobei die genannte Pfropfe auf die Hauptketter durch eine in Tumorzelle spaltbare Bindung verbunden sind.

8. Zusammensetzung nach Anspruch 7, **dadurch gekennzeichnet, dass** das Molekulargewicht der Komponente enthaltend Seitenketten 50 000 bis 400 000 g/mol beträgt.

9. Zusammensetzung nach Ansprüche 7 oder 8, **dadurch gekennzeichnet, dass** die Bindungen verbindend die Hauptkette und die Seitenketten entweder enzymatisch oder reduktiv abbaubar sind.

## Revendications

1. Composition pharmaceutique, possédant une activité anti-tumeur cytotoxique importante et un effet anti-tumeur autostimulant concomitant, avec la doxorubicine comme principe actif, la composition consistant de deux composants, où
a. le premier composant constituant la composition est un copolymère de la N-(2-hydroxypropyl)méthacrylamide (HPMA), marqué PK1, dans lequel la doxorubicine est liée au support polymère par la séquence oligopeptide GlyPheLeuGly clivable par voie enzymatique, où la chaîne polymère consiste de 50-1000 unités monomères consistant de 80-99 % en moles de l'unités N-(2-hydroxypropyl)méthacrylamide (HPMA), 1-5 % en moles de l'oligopeptide méthacryloylaté (GlyPheLeuGly) terminé par la liaison amidique de la doxorubicine liée, 0-10 % en moles de l'oligopeptide méthacryloylaté (GlyPheLeuGly) terminé par le groupe carboxylique, 0-10 % en moles de l'oligopeptide méthacryloylaté (GlyPheLeuGly) terminé par le groupe 2-hydroxypropylamide et éventuellement 0,5-10 % en moles d'unités de l'oligopeptide méthacryloylaté (GlyPheLeuGly) terminées par une molécule d'un anticorps non spécifique de l'homme tel que Hulg, Endobuline, Intraglobine, ou un anticorps monoclonal tel que Erbitux or Herceptine ;
b. le second composant est un copolymère HPMA marqué H, dans lequel la doxorubicine est liée au support polymère par un espaceur contenant une liaison hydrazone sensitive au pH et labile au hydrolyse, qui se compose de 30-3500 unités monomère, reliées en une chaîne polymère linéaire ou ramifiée formée de 60-98,5 % d'unités HPMA, et contient en outre 1-20 % d'unités d'hydrazones méthacryloylatées d'acides α-aminés, d'acides ε-aminés, d'acides aminés aromatiques ou d'oligopeptides terminés par une molécule de la doxorubicine, 0,5-15 % d'unités d'hydrazides d'hydrazones méthacryloylatées d'acides α-aminés, d'acides ε-aminés, d'acides aminés aromatiques ou d'oligopeptides, ou leurs sel de natrium, et éventuellement 0,5-5 % d'unités d'acides α-aminés, d'acides ε-aminés, d'acides aminés aromatiques ou d'oligopeptides terminés par une molécule d'une immunoglobuline ou d'un anticorps monoclonal spécifique méthacryloylatées.

2. Composition selon la revendication 1, **caractérisée en ce que** les composants sont mélangés de telle sorte que la dose résultante de la doxorubicine du premier composant PK1, dans lequel la doxorubicine est liée par une liaison amide, et le second composant H, dans lequel elle est liée par une liaison amide, est dans un rapport en poids PK1/H de 1 : 3 à 3 : 1.

3. Composition selon la revendication 2, **caractérisée en ce que** les composants sont dans un rapport tel que la dose résultante de la doxorubicine PK1/H est dans un rapport en poids de 1 : 2.

4. Composition selon l'une quelconque des revendications précédentes, **caractérisée en ce qu'**au moins l'un des composants est constitué par un système de micelles polymères dont la surface hydrophile est formée par un copolymère HPMA, tandis que le noyau hydrophobe est formé par des hydrocarbures aliphatiques C₉-C₃₀ ou leurs dérivés, ou bien est formé par des hydrocarbures cycliques ou polycycliques C₆-C₃₀.

5. Composition selon la revendication 4, **caractérisée en ce que** la molécule hydrophobe est choisie dans le groupe consistant des acyles d'acides gras C₁₀-C₁₈ ou d'acides gras insaturés, ou d'acides cholique, cholanique (5β-cholane.24-oïque) ou 7-déhydrocholique.

6. Composition selon la revendication 4, **caractérisée en ce que** la molécule hydrophobe est choisie dans le groupe consistant des esters du cholésterol ou du 7-déhydrocholesterol, du cholestanol, de la vitamine D ou des alcools aliphatiques ayant des chaînes en C₉-C₁₈.

7. Composition selon l'une quelconque des revendications 1 à 3, caractérisée en ce la structure d'au moins l'un des composants est constitué par la chaîne principale HPMA portant le principe actif et des chaînes latérales de HPMA-greffes qui peuvent éventuellement aussi porter le principe actif, lesdits greffes étant liées à la chaîne principale par une liaison clivable dans une cellule tumorale.

8. Composition selon la revendication 7, **caractérisée en ce que** le poids moléculaire du composant contenant des chaînes latérales s'élève à 50 000-400 000 g/mole.

9. Composition selon les revendications 7 ou 8, **caractérisée en ce que** les liaisons liant les chaînes principale et latérales sont dégradables, soit par voie enzymatique ou réductive.
